# EUROPEAN PATENT SPECIFICATION

(11) **EP 0 396 137 B1**
(45) Date of publication and mention of the grant of the patent: **19.07.1995**
(21) Application number: 90108379.0
(22) Date of filing: 03.05.1990
(51) Int. Cl.: A61K 7/32, A61K 7/38

(54) **Antiperspirant**
Antiperspirant
Antitranspirant

(30) Priority: 03.05.1989 US 347073
(43) Date of publication of application: 07.11.1990
(73) Proprietor: The Gillette Company, Boston, Massachusetts 02190 (US)
(72) Inventor: Vu, Tuan M., Brighton Massachusetts (US); Krafton, Thomas J., South Hampton New Hampshire (US); Phipps, Alan M., Framingham Massachusetts (US)
(74) Representative: Baillie, Iain Cameron

(56) References cited:
- EP-A- 0 135 315
- DE-A- 2 430 897
- US-A- 3 968 203
- US-A- 4 065 564

## Description

This invention relates to antiperspirant compositions and processes for forming antiperspirant compositions, the compositions being usable as antiperspirant products or for the preparation of such products.

Antiperspirant products are well-known in the cosmetic art. Such antiperspirant products often contain an active antiperspirant ingredient, e.g., aluminum chlorohydrate and a liquid vehicle, Such an active antiperspirant ingredient is soluble in water and water-based antiperspirant products tend to have objectionable characteristics of wetness and tackiness A gelling agent may be used to provide the antiperspirant product with a solid character. Antiperspirant products of the solid-type are typically used by rubbing an area of the body such as the underarm to apply a layer of the antiperspirant to the skin, which reduces perspiration. It is desirable that antiperspirant products have aesthetic characteristics of smoothness, non-oiliness and non-tackiness, other desirable characteristics include clarity of the antiperspirant product and the absence of any readily visible residue as, e.g., a white layer, on the skin after the antiperspirant is applied.

In accordance with the present invention there is provided a substantially clear essentially anhydrous antiperspirant composition comprising an antiperspirant effective amount of an aluminum chlorhydrate or aluminum-zirconium chlorhydrate antiperspirant salt in solid particulate form suspended in an essentially anhydrous vehicle, characterized by said antiperspirant salt being substantially free of opacifying contaminants and the refractive indices of said antiperspirant salt and said vehicle being between 1.46 and 1.56 and substantially matched such that the relative turbidity of said antiperspirant composition is less than 800 FTU. Preferably said refractive indices are matched such that the relative turbidity of the antiperspirant composition is less than 400 FTU.

Also provided in accordance with the present invention is a process for making a substantially clear essentially anhydrous antiperspirant composition characterized by comprising the steps of
providing an aluminum chlorohydrate or aluminum-zirconium chlorohydrate antiperspirant salt in solid particulate form, said antiperspirant salt being substantially free of opacifying contaminants and having a refractive index between 1.46 and 1.56,
providing an essentially anhydrous vehicle with a refractive index between 1.46 and 1.56 that substantially matches the refractive index of said antiperspirant salt such that the relative turbidity of the resulting antiperspirant composition is less than 800 FTU, and
suspending an antiperspirant effective amount of said antiperspirant salt in said vehicle.

US-A-3 968 203 describes an antiperspirant aerosol comprising an astringent salt suspended in fine powder form in an anhydrous liquid vehicle comprising a propellant.

US-A-4 065 564 describes a pump spray or roll-on antiperspirant solution comprising an alcohol soluble aluminum chlorhydroxide complex and a silicone dissolved in an alcohol.

EP-A-0 135 315 concerns a cream comprising an antiperspirant and a gelling agent in a silicone carrier.

In accordance with one aspect of the invention, there Is provided a substantially clear, essentially anhydrous suspension-type antiperspirant product that includes an effective amount of an active antiperspirant component in particulate form suspended in an anhydrous vehicle such that there is no significant dissolution of the active antiperspirant ingredient in the vehicle and the refractive indices of the active antiperspirant component and the vehicle are substantially matched. While particular antiperspirant products include a sufficient quantity of a gelling agent so that the antiperspirant product can be used as a solid, a reduced amount of, or no, gelling agent may be used if it is desirable to make a more liquid type product.

A substantially clear antiperspirant product is one that is visually clear, with a minimal amount of haziness or cloudiness. A transparent antiperspirant solid, like glass, allows ready viewing of objects behind it. By contrast, a translucent antiperspirant solid, although allowing light to pass through, causes the light to be so scattered that it will be impossible to see clearly objects behind the translucent solid. In general, an acceptable translucent to clear antiperspirant product has a turbidity measurement expressed in Nephelometric (or Formazin) Turbidity Units in the range of between about 200 and about 800 FTU, and an antiperspirant product is substantially clear if its relative turbidity measurement is less than 400 FTU. Turbidity measurements discussed hereinafter were made with a Hellige #965 Direct-Reading Turbidimeter. Distilled water has a turbidity of 0 FTU and whole milk diluted one part in 350 parts of distilled water has a turbidity of about 200 FTU.

In accordance with another aspect of the invention, the active antiperspirant component is processed prior to incorporation in the antiperspirant product by gentle removal of water without the formation of opacifying contaminants such as aluminum oxide. For example, if the active antiperspirant component contains a contaminant such as aluminum oxide, an insufficiently clear antiperspirant product can result. In one particular method of processing aluminum chlorohydrate without generating opacifying contaminants for use as the active antiperspirant component, water is removed from an aqueous solution of aluminum chlorohydrate by drying the aqueous solution of aluminum chlorohydrate at a temperature less than 40°C, more preferably less than 30°C. The drying preferably is carried out at atmospheric pressure. The resulting glass-like product is then ground into fine (impalpable) particles (typically less than about one hundred microns in size) for use in the antiperspirant product.

In another particular method, a container is charged with an aluminum chlorohydrate (ACH) solution such as ACH-303 or Rehydrol II, and a surfactant, such as Oleth-10 or Procetyl AWS, is added. The resulting mixture is then agitated to make a clear solution. Ethanol may be added to facilitate dissolution of the surfactant. Solvent (water) or solvents (water and ethanol) are than removed under vacuum (p>10 mmHg) and elevated temperature (T<50°C). Removal of solvent or solvents continues until the mass of the dried solid indicates a predetermined (by calculation) quantity of solvent or solvents has been removed. The dried solid ACH/surfactant adduct is stored in a sealed container.

Antiperspirant components for use in the antiperspirant compositions are well-known aluminum chlorohydrate and aluminum-zirconium chlorohyrate salts, such as are commercially-available as Dow Corning ACH-303 or Rehydrol II (supplied by Reheis Chemical Co.). Further examples of active antiperspirant ingredients that have application to this technology include aluminum sesquichlorohydrate, aluminum dichlorohydrate, aluminum zirconium trichlorohydrate, aluminum zirconium tetrachlorohydrate, aluminum zirconium pentachlorohdyrate and aluminum zirconium octachlorohydrate.

The refractive index of the hydrated active ingredient is a function of the amount of associated water in the solid particulate active ingredient. For example, tray-dried aluminum chlorohydrate has a refractive index η_{D} of about 1.51, and the refractive index of less dried aluminum chlorohydrate (alone or in adduct form) can vary to as low as about 1.46 depending upon the dryness (the amount of water associated with the hydrated salt or adduct). Another useful active ingredient is aluminum-zirconium chlorohydrate that has been dried to have a refractive index η_{D} of about 1.56. The amount of associated water in the solid particulate active ingredient may be adjusted in various ways, including, for example, by controlled drying, by storage of the dried active ingredient in a high humidity atmosphere, or by addition of water to the adduct before addition of a gelling agent.

The active antiperspirant component(s) should be present in an amount effective to reduce perspiration when applied to the skin. The precise limitt on the amount of active antiperspirant component that can be used will vary with the particular component and formula. As a general rule, however, the antiperspirant product should contain anywhere from about 5% to about 50% (more preferably about 10% to about 30%) of active antiperspirant component in solid particulate form.

To provide a substantially clear antiperspirant product, the refractive index (η_{D}) of the vehicle should be matched to that of the active antiperspirant component. The vehicle generally is a blend of liquids having different refractive indexes. Liquids suitable for use include octylmethoxy cinnamate (η_{D} 1.54), isopropyl myristate (η_{D} 1.43), polyphenylmethylsiloxane liquids as Dow Corning DC-556 (η_{D} 1.46), isoeicosane (Permethyl 102A) fluid (η_{D} 1.44), cinnamic aldehyde (η_{D} 1.62), SD-40 alcohol (η_{D} 1.36), C₁₂₋₁₅ alcohols benzoate (Finsolv TN) (η_{D} 1.48), isoamyl methoxy cinnamate (η_{D} 1.56), and isopropyl palmitate (η_{D} 1.48). The vehicle can also include a surfactant, such as Oleth-10 or PPG-5 Ceteth-20 which helps provide washability, provided it is compatible with other requirements of the system. The vehicle can also contain additional cosmetic ingredients such as emollients, colorants, fragrances, and preservatives, again provided that they are compatible and the resulting refractive index of the vehicle is appropriately adjusted.

The components are blended together in appropriate quantities to give a vehicle with an appropriate η_{D}. Most preferably, the η_{D} of the vehicle is virtually identical to that of the active antiperspirant particles. Even when the refractive indices are not identical, however, a substantially clear antiperspirant can be obtained. For example, where the active antiperspirant component is aluminum chlorohydrate (η_{D} is about 1.51), a substantially clear antiperspirant is obtained if the liquid vehicle has an η_{D} between about 1.50 and 1.52; and where the active antiperspirant component is aluminum chlorohydrate (or adduct) with additional associated water (η_{D} is about 1.495), a substantially clear antiperspirant is obtained if the liquid vehicle has an η_{D} between about 1.49 and 1.51. The term "match", as used herein, means the refractive indices are close enough such that an antiperspirant product is obtained that has a turbidity value not in excess of 800 FTU.

The gelling agent used in particular antiperspirant products is compatible with the other components, desirably forms a solution with other liquid components, and, in conjunction with other vehicle components, meets the η_{D} requirements for a substantially clear antiperspirant. Examples of suitable gelling agents include polyethylene-vinyl acetate copolymers, polyethylene homopolymers and blends. Allied Corp's grade 6 and 6A homopolymers and 400 and 400A copolymers are preferred gelling agents. It will be apparent that other appropriate gelling agents with suitably matched refractive indices can be used. The amount of gelling agent in the solid antiperspirant product preferably is sufficient to cause the liquid vehicle to gel (i.e., become free-standing). In general, the solid antiperspirant product should include between about 2% and about 50% (more preferably between about 10% and about 35%, most preferably between about 15% and about 30%) of gelling agent. For liquid antiperspirant products, it may be desirable to use some gelling agent in the antiperspirant product for thickening, even though a sufficient quantity of gelling agent is not used to obtain a free-standing composition.

In accordance with a further aspect of the invention, care is taken in the blending of components to achieve optimum clarity of a gel antiperspirant product. In making a substantially clear essentially anhydrous antiperspirant product, a gelling agent is dissolved in the vehicle component at a temperature of less than the cloud point of the gelling agent; an active antiperspirant component (directly or in adduct form) is added to the gelling agent-vehicle component, the refractive index of the active antiperspirant component matching that of the gelling agent-vehicle component; and the mixture of the gelling agent-vehicle component and the active antiperspirant component is cooled to provide an antiperspirant product that has a relative turbidity of less than 400 FTU.

In preferred embodiments, the active antiperspirant component is aluminum chlorohydrate with a refractive index (depending on the amount of associated water) between about 1.46 and 1.53; and the refractive indices (η_{D}) of the active antiperspirant component and of the gelling agent-vehicle component are within about 0.02. The process may include the step of adding water to the aluminum salt in solid form to reduce its refractive index without significant dissolution of the solid aluminum salt.

In a particular process, an antiperspirant product that includes a polyethylene-vinyl acetate copolymer (AC-400) as a gelling agent is processed as follows: (1) the active antiperspirant component and the polyethylene-vinyl acetate copolymer are added to other vehicle components; (2) the mixture is heated to a temperature below 80°C (preferably between 70°C and 80°C; and most preferably about 75°C) to dissolve the copolymer; and the mixture is cooled.

Although the precise technical reason the above process provides an optimally clear solid antiperspirant product is undetermined, it is felt that keeping the highest processing temperature below the cloud point of the gelling agent (a polyethylene-vinyl) acetate copolymer (such as Allied Corp. AC-400A, which has a η_{D} of about 1.497) generally has a cloud point above 80°C, and a polyethylene homopolymer (such as Allied Corp. AC-6A, which has a η_{D} of about 1.51) generally has a cloud point above 95-100°C) provides the clearest antiperspirant product. Heating above the cloud point may cause a change in the structure of the gelling agent which may change the refractive index and result in a hazy antiperspirant product.

The following Examples 1-12 illustrate representative antiperspirant products and are given by way of illustration only and are not to be considered as being limiting. The amounts in the Examples and the claims are in weight percent.

The following Examples are made by combining and mechanically mixing all the liquid vehicle ingredients until homogeneous and refractive index is adjusted as necessary, for example to 1.5075 where dry aluminum chlorohydrate in adduct form is the antiperspirant product active; to 1.4955 where aluminum chlorohydrate in adduct form contains additional associated water is the antiperspirant product active; and to about 1.56 where aluminum-zirconium chlorohydrate is the antiperspirant product active. The active ingredient is then added, and the mixture is heated from room temperature to 60°C over a period of about one-quarter hour. At 60°C, a gellant (e.g., a polyethylene-vinyl acetate copolymer) (if used) is added and the mixture is continued to be heated to about 75°C (a temperature where the gellant has melted and the mixture appears to be homogeneous). The mixture is held at 75°C for about ten minutes and then deaerated as appropriate and poured into molds.

### Example 1

| Ingredient | Percentage |
|---|---|
| Octylmethoxy Cinnamate | 50.25 |
| Isopropyl myristate | 17.25 |
| Ethanol - 200 proof | 2.50 |
| Aluminum chlorohydrate | 10.00 |
| Polyethylene/Vinyl Acetate Copolymer | 20.00 |

The resulting composition of Example 1 was a solid and had a measured turbidity of 148 FTU.

### Example 2

| Ingredient | Percentage |
|---|---|
| Octylmethoxy Cinnamate | 43.50 |
| Isopropyl myristate | 14.50 |
| Ethanol - 200 proof | 2.50 |
| Aluminum Chlorohydrate | 30.00 adduct form |
| Oleth-10 | 7.50 |
| Polyethylene/Vinyl Acetate Copolymer | 2.00 |

The resulting composition of Example 2 was a viscous fluid and had a measured turbidity of 45 FTU.

### Example 3

| Ingredient | Percentage |
|---|---|
| Octylmethoxy Cinnamate | 39.75 |
| Isopropyl myristate | 12.75 |
| Ethanol - 200 proof | 2.50 |
| Aluminum Chlorohydrate | 20.00 adduct form |
| Oleth-10 | 5.0 |
| Polyethylene/Vinyl Acetate Copolymer | 20.00 |

The resulting composition of Example 3 was a solid and had a measured turbidity of 87 FTU.

### Example 4

| Ingredient | Percentage |
|---|---|
| Octylmethoxy Cinnamate | 34.33 |
| Isopropyl myristate | 10.67 |
| Ethanol - 200 proof | 2.50 |
| Aluminum Chlorohydrate | 30.00 adduct form |
| Oleth-10 | 7.50 |
| Polyethylene/Vinyl Acetate Copolymer | 15.00 |

The resulting composition of Example 4 was a solid and had a turbidity of 102 FTU.

### Example 5

| Ingredient | Percentage |
|---|---|
| Octylmethoxy Cinnamate | 24.50 |
| Finsolv TN | 18.00 |
| Ethanol - 200 proof | 2.50 |
| Aluminum Chlorohydrate | 30.00 adduct form |
| Procetyl AWS | 10.00 |
| Polyethylene/Vinyl Acetate Copolymer | 15.00 |

The resulting composition of Example 5 was a solid and had a turbidity of 127 FTU.

### Example 6

| Ingredient | Percentage |
|---|---|
| Octylmethoxy Cinnamate | 13.53 |
| Finsolv TN | 44.47 |
| Water (added to the adduct) | 2.00 |
| Aluminum Chlorohydrate | 15.00 adduct form |
| Procetyl AWS | 5.00 |
| Polyethylene/Vinyl Acetate Copolymer | 20.00 |

The resulting composition of Example 6 was a substantially clear solid, had a refractive index of 1.4949, and had a turbidity of 234 FTU.

### Example 7

| Ingredient | Percentage |
|---|---|
| Octylmethoxy Cinnamate | 27.50 |
| Finsolv TN | 20.00 |
| Ethanol - 200 proof | 2.50 |
| Aluminum Chlorohydrate | 30.00 adduct form |
| Procetyl AWS | 5.00 |
| Polyethylene/Vinyl Acetate Copolymer | 15.00 |

The resulting composition of Example 7 was a solid and had a turbidity of 100 FTU.

### Example 8

| Ingredient | Percentage |
|---|---|
| Isoamyl Methoxy Cinnamate | 30.89 |
| Isopropyl Myristate | 11.61 |
| Ethanol - 200 proof | 2.50 |
| Isoeicosane | 5.00 |
| Aluminum Chlorohydrate | 20.00 adduct form |
| Oleth-10 | 5.00 |
| Stearyl Alcohol | 2.00 |
| Polyethylene/Vinyl Acetate Copolymer | 23.00 |

The resulting composition of Example 8 was a solid and had a turbidity of 121 FTU.

### Example 9

| Ingredient | Percentage |
|---|---|
| Isoamyl Methoxy Cinnamate | 34.14 |
| Isopropyl Myristate | 8.36 |
| Ethanol - 200 proof | 2.50 |
| Polyphenylmethylsiloxane (DC556) | 5.00 |
| Aluminum Chlorohydrate | 20.00 adduct form |
| Oleth-10 | 5.00 |
| Stearyl Alcohol | 2.00 |
| Polyethylene/Vinyl Acetate Copolymer (AC400A) | 23.00 |

The resulting composition of Example 9 was a solid and had a turbidity of 156 FTU.

### Example 10

| Ingredient | Percentage |
|---|---|
| Octylmethoxy Cinnamate | 33.6 |
| Isopropyl Myristate | 10.4 |
| Ethanol - 200 proof | 2.50 |
| Rehydrol II | 30.00 adduct form |
| Oleth-10 | 7.50 |
| Polyethylene/Vinyl Acetate Copolymer (AC 400A) | 16.00 |

The resulting composition of Example 10 was a solid and had a turbidity Of 56 FTU.

### Example 11

| Ingredient | Percentage |
|---|---|
| Isoamyl Methoxy Cinnamate | 72.3 |
| Cinnamic Aldehyde | 7.7 |
| Aluminum Zirconium Chlorohydrate | 20.00 |

The resulting composition of Example 11 was a liquid and had a turbidity of 450 FTU.

### Example 12

| Ingredient | Percentage |
|---|---|
| Octylmethoxy Cinnamate | 43.10 |
| Isopropyl Myristate | 14.80 |
| Alcohol SD-40 | 2.10 |
| Aluminum Chlorohydrate | 20.00 |
| Polyethylene/Vinyl Acetate Copolymer AC400A | 20.00 |

The resulting composition of Example 12 was a solid and had a turbidity of 290 FTU.

## Claims

1. A substantially clear essentially anhydrous antiperspirant composition comprising an antiperspirant effective amount of an aluminum chlorhydrate or aluminum-zirconium chlorhydrate antiperspirant salt in solid particulate form suspended in an essentially anhydrous vehicle, characterized by said antiperspirant salt being substantially free of opacifying contaminants and the refractive indices of said antiperspirant salt and said vehicle being between 1.46 and 1.56 and substantially matching such that the relative turbidity of said antiperspirant composition is less than 800 FTU.

2. The antiperspirant composition of claim 1 wherein the refractive indices (n_{D}) of said antiperspirant salt and of said vehicle are matched such that the relative turbidity of said antiperspirant composition is less than 400 FTU.

3. The antiperspirant composition of claim 1 or 2 which includes from 10% to 30% of said antiperspirant salt.

4. An antiperspirant composition according to any one of the preceding claims, wherein said vehicle includes a gelling agent having one or more of the following attributes:
(a) said antiperspirant composition includes between 2% and 50% of said gelling agent;
(b) said gelling agent is polyethylene polymer such as a polyethylene-vinyl acetate copolymer;
(c) said gelling agent is present in sufficient quantity to cause said product to be a free-standing solid;
(d) said antiperspirant product includes between 15% and 30% of said gelling agent.

5. A process for making a substantially clear essentially anhydrous antiperspirant composition characterized by comprising the steps of
providing an aluminum chlorohydrate or aluminum-zirconium chlorohydrate antiperspirant salt in solid particulate form, said antiperspirant salt being substantially free of opacifying contaminants and having a refractive index between 1.46 and 1.56,
providing an essentially anhydrous vehicle with a refractive index between 1.46 and 1.56 that substantially matches the refractive index of said antiperspirant salt such that the relative turbidity of the resulting antiperspirant composition is less than 800 FTU, and
suspending an antiperspirant effective amount of said antiperspirant salt in said vehicle.

6. A process according to claim 5, further comprising adding a gelling agent and including one or more of the following:
(a) said antiperspirant composition includes between 2% and 50% or between 10% and 35% of said gelling agent and is a free-standing solid;
(b) said process comprises the further steps of dissolving said gelling agent in said vehicle at a temperature of less than the cloud point of said gelling agent;
(c) the refractive indices (n_{D}) of said active antiperspirant salt and of said vehicle are matched within about 0.02 such that the relative turbidity of the resulting antiperspirant composition is less than 400 FTU; and/or
(d) said gelling agent comprises a polyethylene-vinyl acetate copolymer, and said process further comprises the step of;
dissolving said copolymer in said vehicle at a temperature of less than 80°C;
adding said antiperspirant salt to said copolymer-vehicle solution, the refractive index of said active antiperspirant component matching that of said vehicle; and
cooling the mixture of said vehicle and said antiperspirant salt to provide an antiperspirant composition that has a relative turbidity of less than 800 FTU.

7. A process according to claim 5 or 6, wherein said antiperspirant salt is obtained by the steps of:
providing a solution of said antiperspirant salt and a solvent;
heating said solution at a low temperature, such as to a temperature of less than 50°C, to evaporate a predetermined amount of said solvent and provide said antiperspirant salt in solid form that is substantially free of opacifying contaminants; and
further processing said active antiperspirant salt to provide said antiperspirant salt in solid particulate form.

8. The Process of any one of claims 5 to 7, wherein said solution further contains a surfactant, and said heating step provides an adduct of said antiperspirant salt and said surfactant in solid form that is substantially free of opacifying contaminants.

9. A process according to claim 6, 7 or 8 which comprises the steps of:
dissolving a gelling agent in said vehicle at a temperature of less than the cloud point of said gelling agent;
combining said antiperspirant salt in solid particulate form with said gelling agent and vehicle, the refractive index of said antiperspirant salt matching that of said gelling agent and vehicle; and
cooling the mixture of said gelling agent and vehicle and said antiperspirant salt to provide an antiperspirant composition that has a relative turbidity of less than 400 FTU.

10. A process according to any one of claims 5 to 9, including the step of adding water to said antiperspirant salt in solid form to reduce its refractive index without significant dissolution of said solid active antiperspirant salt.

## Patentansprüche

1. Weitgehend klare, im wesentlichen wasserfreie antihidrotische Zusammensetzung, umfassend eine antihidrotisch wirksame Menge eines Aluminiumchlorhydrats oder Aluminium-Zirconiumchlorhydrats als antihidrotisches Salz in fester, partikulärer Form, suspendiert in einem im wesentlichen wasserfreien Träger, dadurch gekennzeichnet, daß das antihidrotische Salz weitgehend frei von trübenden Verunreinigungen ist und die Brechungsindizes des antihidrotischen Salzes und des Trägers zwischen 1,46 und 1,56 liegen und weitgehend angepaßt sind, so daß die relative Trübung der antihidrotischen Zusammensetzung kleiner ist als 800 FTU.

2. Antihidrotische Zusammensetzung nach Anspruch 1, bei welcher die Brechungsindizes (n_{D}) des antihidrotischen Salzes und des Trägers derart angepaßt sind, daß die relative Trübung der antihidrotischen Zusammensetzung kleiner ist als 400 FTU.

3. Antihidrotische Zusammensetzung nach Anspruch 1 oder 2, welche 10 % ... 30 % des antihidrotischen Salzes umfaßt.

4. Antihidrotische Zusammensetzung nach einem der vorgenannten Ansprüche, bei welcher der Träger ein Gelbildner einschließt, der ein oder mehrere der folgenden Merkmale aufweist:
(a) die antihidrotische Zusammensetzung umfaßt 2 % ... 50 % des Gelbildners;
(b) der Gelbildner ist Polyethylen-Polymer, beispielsweise ein Polyethylen/Vinylacetat-Copolymer;
(c) der Gelbildner liegt in einer ausreichenden Menge vor, um zu bewirken, daß das Produkt ein freistehender Festkörper ist.
(d) das antihidrotische Produkt umfaßt 15 % ... 30 % des Gelbildners.

5. Verfahren zum Herstellen einer weitgehend klaren, im wesentlichen wasserfreien antihidrotischen Zusammensetzung, gekennzeichnet durch die Schritte:
Schaffen eines Aluminiumchlorhydrats oder Aluminium-Zirconiumchlorhydrats als antihidrotisches Salz in fester, partikulärer Form, welches antihidrotische Salz weitgehend frei ist von trübenden Verunreinigungen und einen Brechungsindex von 1,46 ... 1,56 hat,
Schaffen eines im wesentlichen wasserfreien Trägers mit einem Brechungsindex von 1,46 ... 1,56, der weitgehend derart mit dem Brechungsindex des antihidrotischen Salzes angepaßt ist, daß die relative Trübung der resultierenden antihidrotischen Zusammensetzung kleiner ist als 800 FTU, und
Suspendieren einer antihidrotisch wirksamen Menge des antihidrotischen Salzes in dem Träger.

6. Verfahren nach Anspruch 5, ferner umfassend Zusetzen eines Gelbildners und einschließend eines oder mehrere der Folgenden:
(a) die antihidrotische Zusammensetzung umfaßt 2 % ... 50 % oder 10 % ... 35 % des Gelbildners und ist ein freistehender Festkörper;
(b) das Verfahren umfaßt die weiteren Schritte des Auflösens des Gelbildners in dem Träger bei einer niedrigeren Temperatur als der des Cloudpoints des Gelbildners;
(c) die Brechungsindizes (n_{D}) des aktiven antihidrotischen Salzes und des Trägers sind derart innerhalb von etwa 0,02 angepaßt, daß die relative Trübung der resultierenden antihidrotischen Zusammensetzung kleiner ist als 400 FTU; und/oder
(d) der Gelbildner umfaßt ein Polyethylen/Vinylacetat-Copolymer und das Verfahren ferner die Schritte:
Auflösen des Copolymers in dem Träger bei einer Temperatur von weniger als 80 °C;
Zusetzen des antihidrotischen Salzes zu der Copolymer-Träger-Lösung, wobei der Brechungsindex der aktiven antihidrotischen Komponente der des Trägers angepaßt ist; und
Kühlen der Mischung des Trägers und des antihidrotischen Salzes zum Schaffen einer antihidrotischen Zusammensetzung, die eine relative Trübüng von weniger als 800 FTU aufweist.

7. Verfahren nach Anspruch 5 oder 6, bei welchem des antihidrotische Salz erhalten wird durch die Schritte:
Schaffen einer Lösung des antihidrotischen Salzes und eines Lösemittels;
Erhitzen der Lösung bei einer niedrigen Temperatur, wie beispielsweise bei einer Temperatur von weniger als 50 °C, um eine vorbestimmte Menge des Lösemittels zu verdampfen und das antihidrotische Salz in fester Form zu schaffen, das weitgehend frei ist von trübenden Verunreinigungen; und
ferner Behandeln des antihidrotischen Salzes, um das antihidrotische Salz in fester, partikulärer Form zu schaffen.

8. Verfahren nach einem der Ansprüche 5 bis 7, bei welchem die Lösung ferner ein Tensid enthält, und der Schritt der Erhitzens ein Addukt des antihidrotischen Salzes und des Tensids in fester Form schafft, das weitgehend frei ist von trübenden Verunreinigungen.

9. Verfahren nach Anspruch 6, 7 oder 8, welches die Schritte umfaßt:
Auflösen eines Gelbildners in dem Träger bei einer Temperatur unterhalb der des Cloudpoints des Gelbildners;
Vereinigen des antihidrotischen Salzes in fester, partikulärer Form mit dem Gelbildner und Träger, wobei der Brechungsindex des antihidrotischen Salzes dem des Gelbildners und Trägers angepaßt ist; und
Kühlen der Mischung des Gelbildners und Trägers und des antihidrotischen Salzes, um eine antihidrotische Zusammensetzung zu schaffen, die eine relative Trübung von weniger als 400 FTU aufweist.

10. Verfahren nach einem der Ansprüche 5 bis 9, einschließend die Schritte des Zusetzens von Wasser zu dem antihidrotischen Salz in fester Form, um seinen Brechungsindex ohne signifikante Auflösung des festen aktiven antihidrotischen Salzes herabzusetzen.

## Revendications

1. Composition antiperspirante essentiellement anhydre, sensiblement limpide, comprenant une quantité efficace contre la transpiration d'un sel antiperspirant, chlorhydrate d'aluminium ou chlorhydrate d'aluminium-zirconium, sous forme particulaire solide, en suspension dans un véhicule essentiellement anhydre, caractérisée en ce que ledit sel antiperspirant est sensiblement exempt de contaminants opacifiants et que les indices de réfraction dudit sel antiperspirant et dudit véhicule sont compris entre 1,46 et 1,56 et sensiblement harmonisés de sorte que la turbidité relative de ladite composition antiperspirante est inférieure à 800 FTU.

2. Composition antiperspirante selon la revendication 1, dans laquelle les indices de réfraction (n_{D}) dudit sel antiperspirant et dudit véhicule sont harmonisés de telle sorte que la turbidité relative de ladite composition antiperspirante est inférieure à 400 FTU.

3. Composition antiperspirante selon la revendication 1 ou 2, laquelle comprend entre 10 % et 30 % dudit sel antiperspirant.

4. Composition antiperspirante selon l'une quelconque des revendications précédentes, dans laquelle ledit véhicule comprend un agent gélifiant ayant un ou plusieurs des attributs suivants :
(a) ladite composition antiperspirante comprend entre 2 % et 50 % dudit agent gélifiant ;
(b) ledit agent gélifiant est un polymère de polyéthylène tel que le copolymère polyéthylène-acétate de vinyle ;
(c) ledit agent gélifiant est présent en une quantité suffisante pour transformer ledit produit en un solide autoporteur ;
(d) ledit produit antiperspirant comprend entre 15 % et 30 % dudit agent gélifiant.

5. Procédé de préparation d'une composition antiperspirante essentiellement anhydre, sensiblement limpide, caractérisé en ce qu'il comprend les étapes consistant à
fournir un sel antiperspirant, chlorhydrate d'aluminium ou chlorhydrate d'aluminium-zirconium, sous forme particulaire solide, ledit sel antiperspirant étant sensiblement exempt de contaminants opacifiants et ayant un indice de réfraction compris entre 1,46 et 1,56,
fournir un véhicule essentiellement anhydre ayant un indice de réfraction compris entre 1,46 et 1,56, qui s'harmonise sensiblement avec l'indice de réfraction dudit sel antiperspirant de telle sorte que la turbidité relative de la composition antiperspirante obtenue est inférieure à 800 FTU ; et
mettre en suspension une quantité efficace contre la transpiration dudit sel antiperspirant dans ledit véhicule.

6. Procédé selon la revendication 5, comprenant en outre l'addition d'un agent gélifiant et incluant un ou plusieurs des aspects suivants :
(a) ladite composition antiperspirante comprend entre 2 % et 50 % ou entre 10 % et 35 % dudit agent gélifiant et est un solide autoporteur ;
(b) ledit procédé comprend les étapes supplémentaires consistant à dissoudre ledit agent gélifiant dans ledit véhicule à une température inférieure au point de trouble dudit agent gélifiant ;
(c) les indices de réfraction (n_{D}) dudit sel actif antiperspirant et dudit véhicule sont harmonisés dans la limite d'environ 0,02 de telle sorte que la turbidité relative de la composition antiperspirante obtenue est inférieure à 400 FTU et/ou
(d) ledit agent gélifiant comprend un copolymère polyéthylène-acétate de vinyle et ledit procédé comprend en outre les étapes consistant à
dissoudre ledit copolymère dans ledit véhicule à une température inférieure à 80°C ;
ajouter ledit sel antiperspirant à ladite solution copolymère-véhicule, l'indice de réfraction dudit composant actif antiperspirant étant harmonisé avec celui dudit véhicule et
refroidir le mélange dudit véhicule et dudit sel antiperspirant pour obtenir une composition antiperspirante ayant une turbidité relative inférieure à 800 FTU.

7. Procédé selon la revendication 5 ou 6, dans lequel ledit sel antiperspirant est obtenu par les étapes consistant à :
fournir une solution dudit sel antiperspirant et un solvant ;
chauffer ladite solution à une température faible, par exemple à une température inférieure à 50°C, pour évaporer une quantité prédéterminée dudit solvant et fournir ledit sel antiperspirant sous une forme solide, sensiblement exempte de contaminants opacifiants et
poursuivre le traitement dudit sel actif antiperspirant pour fournir ledit sel antiperspirant sous une forme particulaire solide.

8. Procédé selon l'une quelconque des revendications 5 à 7, dans lequel ladite solution contient en outre un tensioactif et ladite étape de chauffage fournit un produit d'addition dudit sel antiperspirant et dudit tensioactif sous forme solide, lequel est sensiblement exempt de contaminants opacifiants.

9. Procédé selon la revendication 6, 7 ou 8, lequel comprend les étapes consistant à :
dissoudre un agent gélifiant dans ledit véhicule à une température inférieure au point de trouble dudit agent gélifiant ;
associer ledit sel antiperspirant sous forme particulaire solide auxdits agent gélifiant et véhicule, l'indice de rétraction dudit sel antiperspirant étant harmonisé avec celui desdits agent gélifiant et véhicule et
refroidir le mélange desdits agent gélifiant et véhicule et dudit sel antiperspirant pour fournir une composition antiperspirante présentant une turbidité relative inférieure à 400 FTU.

10. Procédé selon l'une quelconque des revendications 5 à 9, comprenant l'étape consistant à ajouter de l'eau audit sel antiperspirant sous forme solide pour en abaisser l'indice de réfraction, en l'absence de dissolution significative dudit sel actif antiperspirant solide.
